# EUROPÄISCHE PATENTSCHRIFT

(11) **EP 1 358 140 B1**
(45) Veröffentlichungstag und Bekanntmachung des Hinweises auf die Patenterteilung: **29.12.2004**
(21) Anmeldenummer: 02708301.3
(22) Anmeldetag: 18.01.2002
(51) Int. Cl.: C07C 11/02

(54) **VERFAHREN ZUR HERSTELLUNG VON 1-OCTEN**
METHOD FOR PRODUCING 1-OCTEN
PROCEDE DE FABRICATION DE 1-OCTENE

(30) Priorität: 08.02.2001 DE 10105751
(43) Veröffentlichungstag der Anmeldung: 05.11.2003
(73) Patentinhaber: Oxeno Olefinchemie GmbH, 45772 Marl (DE)
(72) Erfinder: RÖTTGER, Dirk, 45657 Recklinghausen (DE); TUCHLENSKI, Axel, 45478 Mülheim (DE)
(86) Internationale Anmeldenummer: PCT/EP2002/000455
(87) Internationale Veröffentlichungsnummer: WO 2002/062732

(56) Entgegenhaltungen:
- WO-A-92/10450

## Beschreibung

Die Erfindung betrifft ein Verfahren zur Herstellung von 1-Octen durch Telomerisation von 1,3-Butadien mit einem Telogen in Gegenwart eines Telomerisationskatalysators, partielle Hydrierung des Telomers und Spaltung des hydrierten Zwischenproduktes.

1-Octen wird in großen Mengen in der Produktion verschiedener chemischer Produkte eingesetzt. Beispielsweise werden oberflächenaktive Stoffe, Weichmacher, Schmierstoffe und Polymere aus 1-Octen hergestellt. Ein großes Einsatzgebiet ist weiterhin die Verwendung als Comonomer in Polymeren, insbesondere in Polyethylen.
Nahezu alle zur Zeit kommerziell genutzten Verfahren zur Produktion von 1-Octen basieren auf dem Rohstoff Ethen. Ethen wird oligomerisiert und man erhält ein Produktspektrum von α-Olefinen als Hauptprodukte. Bei passender Wahl von Katalysator und Prozessbedingungen kann die Menge an 1-Octen im Produkt optimiert werden und liegt dann bei ca. 25 %. Neben diesen Verfahren, mit denen die Hauptmenge an 1-Octen produziert wird, hat die Isolierung des 1-Octens aus dem Produktspektrum der Fischer-Tropsch-Reaktion eine gewisse Bedeutung erlangt.

In der Literatur sind neben den auf Ethen basierenden Prozessen auch Verfahren bekannt, die 1,3-Butadien als Rohstoff einsetzen. 1-Octen ist aber nicht direkt, beispielsweise über eine Dimerisierung, aus Butadien erhältlich, sondern wird nach mehreren Prozessschritten erhalten. So beschreibt die Patentanmeldung WO 92/10450 ein Verfahren, bei dem 1,3-Butadien vorzugsweise mit Methanol oder Ethanol zu einem 2,7-Octadienylether umgesetzt wird, der nach Hydrierung zum Octylether zum 1-Octen gespalten wird. In EP-A-0 440 995 wird ein analoger Weg beschritten, die Umsetzung erfolgt im ersten Schritt aber mit einer Carbonsäure. Gemeinsam ist den Verfahren der erste Prozessschritt, den man allgemein als Telomerisation bezeichnet. Bei der Telomerisation wird allgemein ein Telogen (in EP-A-0 440 995 die Carbonsäure) mit einem Taxogen (1,3-Butadien, 2 Äquivalente) zu einem Telomer umgesetzt.

Beispiele für Telomerisationsreaktionen werden unter anderem beschrieben in E. J. Smutny, J. Am. Chem. Soc. 1967, 89, 6793; S. Takahashi, T. Shibano, N. Hagihara, Tetrahedron Lett. 1967, 2451; EP-A-0 561 779, US 3 499 042, US 3 530 187, GB 1 178 812, NL 6 816 008, GB 1 248 593, US 3 670 029, US 3 670 032, US 3 769 352, US 3 887 627, GB 1 354 507, DE 20 40 708, US 4 142 060, US 4 146 738, US 4 196 135, GB 1 535 718, US 4 104 471, DE 21 61 750 und EP-A-0 218 100.

Bei den Verfahren zur Darstellung von 1-Octen auf der Basis von Butadien, wie beispielweise in WO 92/10450 oder EP-A-0 440 995 beschrieben, wird das 1-Octen durch Spaltung eines an 1-Position substituierten n-Octans erhalten. Die Selektivitäten in diesem Schritt sind dabei oftmals unbefriedigend. So wird in WO 92/10450 bei der Spaltung von 1-Methoxyoctan bei einem Umsatz von 80 % eine Selektivität zu Octenen von 66 % genannt.

Es bestand daher die Aufgabe, ein Verfahren aufzufinden, über das 1-Octen auf der Basis von 1,3-Butadien hergestellt werden kann, das aber den oben genannten Spaltungsschritt umgeht.

Es wurde nun gefunden, dass 1-Octen in hoher Reinheit und guter Ausbeute über einen Prozess dargestellt werden kann, der sich im Wesentlichen aus drei Schritten zusammensetzt. Gegenstand der Erfindung ist daher ein Verfahren zur Herstellung von 1-Octen durch
1) Umsetzung von 1,3-Butadien in Gegenwart eines Telomerisationskatalysators mit einem Telogen der allgemeinen Formel I, 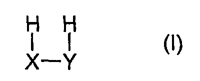 worin X für O, N, S oder P und Y für C, N oder Si steht und wobei X und Y je nach Wertigkeit von X und Y weitere Substituenten tragen können,
   zu einem Telomer der allgemeinen Formel II mit den für X und Y genannten Bedeutungen 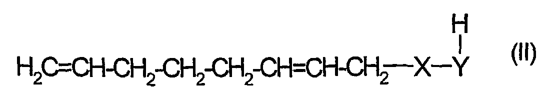
2) partielle Hydrierung der Verbindung gemäß Formel II zur Verbindung gemäß Formel III, 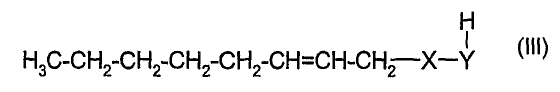 und
3) Gewinnung von 1-Octen durch Spaltung der Verbindung gemäß Formel III.

Für den Telomerisationsprozess in Schritt 1) der vorliegenden Erfindung können sowohl reines 1,3-Butadien als auch Mischungen, die 1,3-Butadien enthalten, eingesetzt werden. Als 1,3-Butadien enthaltende Mischungen kommen vorzugsweise Mischungen von 1,3-Butadien mit anderen C₄-Kohlenwasserstoffen zum Einsatz. Solche Mischungen fallen beispielsweise bei Spalt(Crack)-Prozessen zur Produktion von Ethen an, in denen Raffineriegase, Naphtha, Gasöl, LPG (liquified petroleum gas), NGL (natural gas liquid) usw. umgesetzt werden. Die bei diesen Prozessen als Nebenprodukt anfallenden C₄-Schnitte enthalten je nach Spalt-Verfahren unterschiedliche Mengen an 1,3-Butadien. Typische 1,3-Butadienkonzentrationen im C₄-Schnitt, wie sie aus einem Naphtha-Steamcracker erhalten werden, liegen bei 20 - 70 % 1,3-Butadien.

Die C₄-Komponenten n-Butan, i-Butan, 1-Buten, cis-2-Buten, trans-2-Buten und i-Buten, die ebenfalls in diesen Schnitten enthalten sind, stören die Umsetzung im Telomerisationsschritt nicht oder nicht wesentlich. Diene mit kumulierten Doppelbindungen (1,2-Butadien, Allen usw.) und Alkine, insbesondere Vinylacetylen, wirken hingegen als Moderatoren in der Telomerisationsreaktion. Es ist daher vorteilhaft, die C₄-Alkine und gegebenenfalls das 1,2-Butadien vorher zu entfernen. Dies kann, falls möglich, über physikalische Verfahren wie Destillation oder Extraktion erfolgen. Auf chemischem Wege können die Alkine über Selektivhydrierungen zu Alkenen oder Alkanen und die kumulierten Diene zu Monoenen reduziert werden. Verfahren für derartige Hydrierungen sind Stand der Technik und zum Beispiel in WO 98/12160, EP-A-0 273 900, DE-A-37 44 086 oder US 4 704 492 beschrieben.

Als Telogene können in Schritt 1 des erfindungsgemäßen Verfahrens alle Verbindungen eingesetzt werden, die der allgemeinen Formel I genügen. In Formel I steht X für O, N, S oder P und Y für C, N oder Si, wobei X und Y je nach Wertigkeit von X und Y weitere Substituenten tragen können. Bevorzugte Substituenten an X und Y sind Wasserstoff, Alkylreste mit 1 - 50 Kohlenstoffatomen, Arylreste mit 6 - 50 Kohlenstoffatomen und/oder Heteroarylreste, wobei diese Substituenten jeweils gleich oder verschieden sein können und ihrerseits wiederum mit den Gruppen Alkyl, Aryl, -F, -Cl, -Br, -I, -CF₃, -OR, -COR, -CO₂R, -OCOR, -SR, -SO₂R, -SOR, -SO₃R, -SO₂NR₂, -NR₂, -N=CR₂, -NH₂, mit R = H, substituierter oder unsubstituierter, aliphatischer oder aromatischer Kohlenwasserstoffrest mit 1 bis 25 Kohlenstoffatomen, substituiert sein können. Bevorzugt steht X für O oder N und Y für C. Konkrete Beispiele für Telogene nach der allgemeinen Formel I sind
- Monoalkohole wie zum Beispiel Methanol, Ethanol, n-Propanol, Isopropanol, Allylalkohol, n-Butanol, i-Butanol, Octanol, 2-Ethylhexanol, Isononanol, Benzylalkohol, Cyclohexanol, Cyclopentanol oder 2,7-Octadien-1-ol
- Dialkohole wie zum Beispiel Ethylenglykol, 1,2-Propandiol, 1,3-Propandiol, 1,4-Butandiol, 1,2-Butandiol, 2,3-Butandiol und 1,3-Butandiol
- Hydroxyverbindungen wie zum Beispiel α-Hydroxyessigsäureester
- primäre Amine wie zum Beispiel Methylamin, Ethylamin, Propylamin, Butylamin, Octylamin, 2,7-Octadienylamin, Dodecylamin, Ethylendiamin oder Hexamethylendiamin
- sekundäre Amine wie Dimethylamin, Diethylamin, N-Methylanilin, Bis(2,7-Octadienyl)amin, Dicyclohexylamin, Methylcyclohexylamin, Pyrrolidin, Piperidin, Morpholin, Piperazin oder Hexamethylenimin.

Telogene, die selbst über eine Telomerisationsreaktion erhalten werden können, können direkt eingesetzt oder aber in situ gebildet werden. So kann beispielsweise 2,7-Octadien-1-ol aus Wasser und Butadien in Anwesenheit des Telomerisationskatalysators in situ gebildet werden, 2,7-Octadienylamin aus Ammoniak und 1,3-Butadien usw.

Besonders bevorzugt eingesetzte Telogene sind Methanol, Ethanol, n-Butanol, Ethylenglykol, 1,3-Propandiol, Dimethylamin und Diethylamin. Ganz besonders bevorzugt wird Methanol eingesetzt.

Für das Verhältnis von Telogen zu 1,3-Butadien in der Telomerisationsreaktion ist die Anzahl der aktiven Wasserstoffatome im Telogen zu berücksichtigen. So hat beispielsweise Methanol ein aktives Wasserstoffatom, Ethylenglykol hat zwei, Methylamin hat zwei usw.

Pro Mol aktivem Wasserstoffatom des Telogens, das mit dem 1,3-Butadien reagieren kann, werden 0,001 Mol bis 10 Mol 1,3-Butadien in der Telomerisationsreaktion eingesetzt. Bei einer Reaktionsführung mit einer flüssigen Phase wird ein Verhältnis von 0,1 Mol bis 2 Mol 1,3-Butadien pro Mol aktivem Wasserstoff bevorzugt.

Als Telomerisationskatalysatoren können homogene, heterogene oder immobilisierte Katalysatoren oder Kombinationen hiervon verwendet werden. In der Literatur ist eine Vielzahl von Katalysatoren für diese Reaktion beschrieben (vgl. A. Behr, "Homogeneous Transition Metal Catalysts", Aspects of Homogeneous Catalysis, 1984, 5, 3 - 73). Beispielsweise werden Übergangsmetalle aus der VIII. Nebengruppe des Periodensystems der Elemente und ihre Komplexe erfolgreich als Katalysatoren eingesetzt.

Im Rahmen dieser Erfindung wird der Einsatz von Nickel-, Rhodium-, Palladium- und Platinkatalysatoren bevorzugt. Besonders bevorzugt wird dabei der Einsatz von Palladiumkatalysatoren. Es können sowohl Palladium(0)- als auch Palladium(II)-Verbindungen im Telomerisationsschritt eingesetzt werden. Beispiele für geeignete Palladiumverbindungen sind Palladium(II)-chlorid, Palladium(II)-bromid, Palladium(II)-acetat, Palladium(II)-formiat, Palladium(II)-octanoat, Palladium(II)-carbonat, Palladium(II)-sulfat, Palladium(II)-nitrat, Palladium(II)-acetylacetonat, Palladium(II)-alkylsulfonate, Na₂PdCl₄, K₂PdCl₄, Dichlorobis(benzonitril)palladium, Allylpalladiumchlorid, Allylpalladiumacetat, Trisallylpalladium, 1,5-Cyclooctadienpalladium(II)-chlorid, Bis(triphenylphosphin)palladium(II)-chlorid, (1,2-Bis(diphenylphosphino)ethan)palladium(II)-chlorid. Bei Einsatz von Palladiumhalogeniden muss der Reaktion ein Aktivator zugesetzt werden, da freie Halogenidionen die Telomerisationsreaktion inhibieren. Der Einsatz von Palladium(II)-salzen mit organischen Resten wie Palladiumacetat oder Palladiumacetylacetonat wird daher bevorzugt. Beispiele für Palladium(0)-komplexe umfassen Komplexe des Palladiums mit Phosphor-, Stickstoff- oder Arsendonoratomen, Alkin-, Alken-, und Dienkomplexe. Beispiele für Phosphorliganden sind Phosphine, Phosphite, Phosphonite oder Phosphinite, Beispiele für Stickstoffliganden sind Amine, Nitrile und Pyridine. Konkrete Beispiele sind Tetrakis(triphenylphosphin)palladium(0), Tris(dibenzylidenaceton)dipalladium(0) und Bis(1,5-cyclooctadien)palladium.

Die eingesetzte Menge an Telomerisationskatalysator ist abhängig von seiner Aktivität. Prinzipiell kann jede Katalysatormenge eingesetzt werden, die eine ausreichende Reaktionsgeschwindigkeit gewährleistet. In homogenkatalysierten Reaktionen, bei denen Edukte, Produkte und ein Übergangsmetallkatalysator gelöst in einer Phase vorliegen, werden in der Regel zwischen 0,1 ppm und 50 000 ppm Metall (bezogen auf die Reaktionsmischung) eingesetzt. Bei Einsatz von Palladiumkatalysatoren werden bevorzugt zwischen 1 ppm und 1 000 ppm, besonders bevorzugt zwischen 3 ppm und 100 ppm Katalysatormetall eingesetzt.

Wird die Telomerisation in mehrphasigen Systemen durchgeführt (zum Beispiel heterogenkatalysiert oder zwei flüssige Phasen, von denen eine den Katalysator enthält), können sich diese Konzentrationsbereiche verschieben. Bei der Telomerisation in mehreren flüssigen Phasen ist es besonders vorteilhaft, wenn Katalysator und Produkt in unterschiedlichen Phasen vorliegen, da dann der Katalysator einfach über eine Phasentrennung abgetrennt werden kann. Oftmals bildet dabei Wasser eine der flüssigen Phasen aus. Es werden beispielsweise aber auch perfluorierte Kohlenwasserstoffe, ionische Flüssigkeiten und überkritisches Kohlendioxid eingesetzt (zu ionischen Flüssigkeiten vgl. P. Wasserscheid, W. Keim, Angew. Chem., Int. Ed. 2000, 39, 3772 - 3789). Die Telomerisation von Butadien mit Wasser in ionischen Flüssigkeiten beschreiben J. E. L. Dullius, P. A. Z. Suarez, S. Einloft, R. F. de Souza, J. Dupont, J. Fischer, A. D. Cian, Organometallics 1999, 17, 997 - 1000. Einen Überblick über Wasser als Trägerphase für den Katalysator findet man beispielsweise in B. Comils, W. A. Herrmann (Eds.) "Aqueous-Phase Organometallic Catalysis ", Wiley-VCH, Weinheim, New-York, Chichester, Brisbane, Singapore, Toronto, 1998. Besonders vorteilhaft ist es bei Verfahren mit mehreren flüssigen Phasen ein Telogen einzusetzen, das zusammen mit dem Katalysator in einer Phase vorliegt, die Produkte sich aber hauptsächlich in einer zweiten Phase befinden.

Die Telomerisationskatalysatoren können in aktiver Form in den Prozess eingebracht werden. Oft ist es jedoch einfacher, einen Vorläufer (Precursor) einzusetzen, der unter den Reaktionsbedingungen die katalytisch aktive Spezies ausbildet.

Durch Zugabe von Liganden zur Telomerisationsreaktion kann der Reaktionsverlauf in der Regel erheblich verbessert werden. Es ist daher von Vorteil, Schritt 1 des erfindungsgemäßen Verfahrens in Gegenwart von Liganden durchzuführen. Prinzipiell sind alle Liganden geeignet, die die Reaktionsgeschwindigkeit erhöhen, die Selektivität der Bildung von Verbindung II verbessern, die Katalysatorstandzeit verlängern usw. Beispiele für geeignete Liganden sind Verbindungen mit einem oder mehreren trivalenten Phosphor-, Arsen-, Antimon- oder Stickstoffatomen.

### Beispiele für Phosphorliganden sind:

Phosphine wie zum Beispiel Triphenylphosphin, Tris(p-tolyl)phosphin, Tris(m-tolyl)phosphin, Tris(o-tolyl)phosphin, Tris(p-methoxyphenyl)phosphin, Tris(p-dimethylaminophenyl)phosphin, Tricyclohexylphosphin, Tricyclopentylphosphin, Triethylphosphin, Tris(1-naphthyl)phosphin, Tribenzylphosphin, Tri-n-butylphosphin, Tri-tert.-butylphosphin, Tris(3-sulfonato-phenyl)-phosphin (Metallsalz), Bis(3-sulfonato-phenyl)phenylphosphin (Metallsalz), (3-sulfonato-phenyl)diphenylphosphin (Metallsalz),
Phosphite wie zum Beispiel Trimethylphosphit, Triethylphosphit, Tri-n-propylphosphit, Tri-i-propylphosphit, Tri-n-butylphosphit, Tri-i-butylphosphit, Tri-tert.-butylphosphit, Tris(2-ethylhexyl)phosphit, Triphenylphosphit, Tris(2,4-di-tert.-butylphenyl)phosphit, Tris(2-tert.-butyl-4-methoxyphenyl)phosphit, Tris(2-tert.-butyl-4-methylphenyl)phosphit, Tris(p-kresyl)phosphit, Phosphonite wie zum Beispiel Methyldiethoxyphosphin, Phenyldimethoxyphosphin, Phenyldiphenoxyphosphin, 2-Phenoxy-2H-dibenz[c,e][1,2]oxaphosphorin und dessen Derivate, in denen die Wasserstoffatome ganz oder teilweise durch Alkyl- und/oder Arylreste oder Halogenatome ersetzt sind,
Phosphinite wie zum Beispiel Diphenyl(phenoxy)phosphin und dessen Derivate, in denen die Wasserstoffatome ganz oder teilweise durch Alkyl- und/oder Arylreste oder Halogenatome ersetzt sind, Diphenyl(methoxy)phosphin, Diphenyl(ethoxy)phosphin usw.

Im Rahmen dieser Erfindung werden auch Phosphoniumsalze als Liganden verstanden. Beispiele für geeignete Phosphoniumsalze und ihren Einsatz in der Telomerisation finden sich unter anderem in EP-A-0 296 550.

Bei Einsatz von Übergangsmetallkatalysatoren beträgt das Verhältnis von Ligand zu Metall (Mol/Mol) normalerweise 0,1/1 bis 500/1, bevorzugt 0,5/1 bis 50/1, besonders bevorzugt 1/1 bis 20/1. Der Ligand kann der Reaktion in Substanz, gelöst oder in Form von Metallkomplexen zugeführt werden. Zusätzlicher Ligand kann der Reaktion zu jedem Zeitpunkt und an beliebiger Stelle im Reaktor in Substanz, als Lösung oder in Form eines Metallkomplexes zugeführt werden.

Oftmals ist es vorteilhaft, die Telomerisationsreaktion in Gegenwart von Basen durchzuführen. Beispiele für geeignete Basen sind Metallhydroxide, insbesondere Alkalimetallhydroxide und Erdalkalimetallhydroxide, Metallcarbonate und Metallhydrogencarbonate, insbesondere Alkalimetall- und Erdalkalimetallcarbonate und Alkali- und Erdalkalimetallhydrogencarbonate, Hydroxide quartärer Ammonium- oder Phosphoniumionen, Alkoholate, Alkoxide, Enolate, Phenolate, Metallsalze von Carbonsäuren, Metallamide wie zum Beispiel Natriumamid oder Lithiumdiethylamid, Alkalimetallborhydride, Alkalimetallaluminiumhydride und organische Stickstoffbasen, insbesondere Amine wie zum Beispiel Triethylamin, Pyridin oder Trioctylamin.
Besonders vorteilhaft ist es, Metallsalze des Telogens entsprechend der allgemeinen Formel IV einzusetzen.

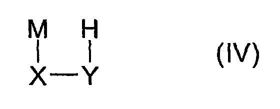

Darin bedeutet M ein ein oder entsprechend der Stöchiometrie anteilig mehrwertiges Metall. X und Y haben die bereits angegebenen Bedeutungen. Bevorzugt steht M für ein Alkalimetall, Erdalkalimetall, Bor oder Aluminium, besonders bevorzugt für Lithium, Natrium oder Kalium. Verbindungen nach der allgemeinen Formel IV können oftmals einfach aus der Reaktion des Telogens nach Formel I mit dem Metall erhalten werden. Dies kann auch in situ erfolgen.

Die Menge an eingesetzter Base, die der Telomerisationsreaktion zugesetzt wird, ist stark abhängig von der Art der eingesetzten Base. Bei Einsatz von Übergangsmetallkatalysatoren werden normalerweise 0 bis 50 000 Mol Base pro Mol Übergangsmetall eingesetzt, bevorzugt 0,5 bis 5 000, besonders bevorzugt 0,5 bis 500 Mol Base pro Mol Übergangsmetall. Es ist auch möglich, mehrere Basen auf einmal einzusetzen.

Für die Durchführung von Schritt 1 des erfindungsgemäßen Verfahrens kann der Zusatz anderer Hilfsstoffe Vorteile bringen, beispielsweise der Einsatz von Inhibitoren, die die Polymerisation des Butadiens unterdrücken. Derartige Inhibitoren sind normalerweise auch im kommerziellen, (stabilisierten) reinen 1,3-Butadien enthalten. Ein Standardstabilisator ist beispielsweise tert.-Butylbrenzkatechol.

Schritt 1 des erfindungsgemäßen Verfahrens kann ohne Lösemittel oder unter Zusatz von Lösemitteln durchgeführt werden. Die eingesetzten Lösemittel sollten dabei weitgehend inert sein. Bevorzugt wird der Zusatz von Lösemitteln bei Einsatz von Telogenen, die unter den Reaktionsbedingungen als Feststoffe vorliegen oder bei Produkten, die unter den Reaktionsbedingungen als Feststoffe anfallen würden. Geeignete Lösemittel sind unter anderem aliphatische, cycloaliphatische und aromatische Kohlenwasserstoffe wie zum Beispiel C₃-C₂₀-Alkane, Mischungen niederer oder höherer Alkane (C₃-C₂₀), Cyclohexan, Cyclooctan, Ethylcyclohexan, Alkene und Polyene, Vinylcyclohexen, 1,3,7-Octatrien, die C₄-Kohlenwasserstoffe aus Crack-C₄-Schnitten, Benzol, Toluol und Xylol; polare Lösemittel wie zum Beispiel tertiäre und sekundäre Alkohole, Amide wie zum Beispiel Acetamid, Dimethylacetamid und Dimethylformamid, Nitrile wie zum Beispiel Acetonitril und Benzonitril, Ketone wie zum Beispiel Aceton, Methylisobutylketon und Diethylketon, Carbonssäureester wie zum Beispiel Essigsäureethylester, Ether wie beispielsweise Dipropylether, Diethylether, Methyl-tert.-butylether (MTBE), Dimethylether, Methyloctylether, 3-Methoxyoctan, Dioxan, Tetrahydrofuran, Anisol, Alkyl- und Arylether von Ethylenglykol, Diethylenglykol und Polyethylenglykol und andere polare Lösemittel wie zum Beispiel Sulfolan, Dimethylsulfoxid, Ethylencarbonat und Propylencarbonat. Auch Wasser kann als Lösemittel eingesetzt werden. Die Lösemittel kommen allein oder als Mischungen verschiedener Lösemittel zum Einsatz.

Schritt 1 der vorliegenden Erfindung wird vorteilhaft unter Ausschluss von Sauerstoff durchgeführt, da Sauerstoff sich nachteilig auf die Stabilität der Katalysatorsysteme auswirkt.

Die Temperatur, bei der die Telomerisationsreaktion ausgeführt wird, liegt zwischen 10 °C und 200 °C, bevorzugt zwischen 40 °C und 150°C, besonders bevorzugt zwischen 40 °C und 110 °C. Der Reaktionsdruck beträgt 1 bar bis 300 bar, bevorzugt 1 bar bis 120 bar, besonders bevorzugt 1 bar bis 64 bar und ganz besonders bevorzugt 1 bar bis 20 bar.

Für das erfindungsgemäße Verfahren ist es nicht notwendig, einen vollständigen Umsatz des Butadiens in der Telomerisation zu erreichen. Der Umsatz des Butadiens liegt zwischen 5 % und 100 %, bevorzugt zwischen 50 % und 100 %, besonders bevorzugt zwischen 80 % und 100%.

Schritt 1 des erfindungsgemäßen Verfahrens kann kontinuierlich oder diskontinuierlich betrieben werden und ist nicht auf den Einsatz bestimmter Reaktortypen begrenzt. Beispiele für Reaktoren, in denen die Reaktion durchgeführt werden kann, sind Rührkesselreaktor, Rührkesselkaskade, Strömungsrohr und Schlaufenreaktor. Auch Kombinationen verschiedener Reaktoren sind möglich, beispielsweise ein Rührkesselreaktor mit nachgeschaltetem Strömungsrohr.

Die bei der Reaktion entstehende Reaktionswärme wird nach bekannten Verfahren abgeführt, beispielsweise durch interne oder externe Kühler. Konkret kann dies den Einsatz von Rohrbündelreaktoren, Reaktoren mit Kühlfingern, Kühlschlangen oder -platten oder die Kühlung eines Rückführungsstroms (Reaktoren mit Kreislauf, Recycling), bedeuten.

Der in Stufe 1 des erfindungsgemäßen Verfahrens eingesetzte Telomerisationskatalysator kann nach der Telomerisationsreaktion zurückgewonnen und ganz oder teilweise für weitere Telomerisationsreaktionen eingesetzt werden (vgl. EP-A-0 218 100). Die Abtrennung des Katalysators kann beispielsweise über eine Destillation, Extraktion, Fällung oder Adsorption erfolgen. Liegt der Katalysator ganz oder teilweise in einer zweiten Phase vor, kann die Trennung einfach durch Trennung der Phasen erfolgen.

Es ist auch möglich, dass der Katalysator vor der Abtrennung oder während der Abtrennung modifiziert wird. Dies gilt analog für die vollständige oder teilweise Rückführung in den Prozess, der ebenfalls eine Modifikation des Katalysators vorgeschaltet werden kann. Beispielsweise wird in US 4 146 738 ein Verfahren beschrieben, bei dem vor der Katalysatorabtrennung der Katalysator durch Hilfsstoffe stabilisiert wird. Nach der Trennung von den anderen Produkten erfolgt eine Aktivierung und Rückführung in den Prozess.

Alternativ kann der Katalysator nach der Reaktion auch anderweitig aufgearbeitet werden (vgl. WO 90/13531, US 5 254 782).

Wird in Schritt I das eingesetzte Telogen nicht vollständig umgesetzt, wird das überschüssige Telogen bevorzugt vom Austrag aus Schritt I des erfindungsgemäßen Verfahrens abgetrennt und ganz oder teilweise in Schritt I zurückgeführt.

In Schritt 1 des erfindungsgemäßen Verfahrens werden neben dem Produkt nach der allgemeinen Formel II als Nebenprodukte hauptsächlich an der 3-Position substituiertes 1,7-Octadien, 1,3,7-Octatrien und 4-Vinylcyclohexen erhalten. Hinzu kommen geringe Mengen an höhersiedenden Komponenten. Für das weitere Verfahren kann es vorteilhaft sein, die Nebenprodukte ganz oder teilweise vom Produkt nach der allgemeinen Formel II abzutrennen. Grundsätzlich können alle Verfahren oder Kombinationen von Verfahren zu Anwendung kommen, mit denen die Verbindung nach der allgemeinen Formel II aus dem Produktgemisch abgetrennt werden kann. Bevorzugte Trenntechnik ist die Destillation. Für die destillative Trennung können alle verfügbaren Techniken eingesetzt werden, beispielsweise Bodenkolonnen, Kolonnen mit Packungen, Trennwandkolonnen, Extraktivdestillation, Dünnschichtverdampfer und Fallfilmverdampfer. Die destillative Trennung kann in einem oder in mehreren Schritten erfolgen und ist abhängig von den Siedepunkten der im Produktgemisch enthaltenen Komponenten. Werden butadienhaltige Mischungen von C₄-Kohlenwasserstoffen als Edukte eingesetzt, haben die verbleibenden C₄-Kohlenwasserstoffe den niedrigsten Siedepunkt und können daher einfach über Kopf abgetrennt werden.

Wenn in den verbleibenden C₄-Kohlenwasserstoffen Isobuten enthalten ist und als Telogen Alkohole eingesetzt werden, ergibt sich zudem die Möglichkeit, überschüssigen Alkohol zusammen mit den C₄-Kohlenwasserstoffen abzutrennen und in anderen Prozessen weiter umzusetzen. Ist beispielsweise Isobuten in den C₄-Kohlenwasserstoffen enthalten und es wird Methanol als Telogen eingesetzt, so können nach der Telomerisation verbleibende C₄-Kohlenwasserstoffe zusammen mit überschüssigem Methanol abgetrennt und gemeinsam in eine MTBE Synthese eingespeist werden.

Es kann weiterhin von Vorteil sein, andere Komponenten des Austrags aus Schritt 1 des erfindungsgemäßen Verfahrens zu isolieren und gegebenenfalls in den Prozess zurückzuführen oder separat zu verwerten. Für die hierzu eingesetzten Techniken gilt analog dasselbe wie bei der Isolierung des Produkts nach der allgemeinen Formel II. Als zu isolierende Komponenten eignen sich insbesondere das eingesetzte Telogen, überschüssiges 1,3-Butadien, das an 3-Position substituierte 1,7-Octadien, 1,3,7-Octatrien, 4-Vinylcyclohexen, die eingesetzte Base beziehungsweise die eingesetzten Basen und gegebenenfalls eingesetzte Lösemittel.

Das Produkt nach der allgemeinen Formel II wird in Schritt 2 zum Produkt nach der allgemeinen Formel III hydriert. Das Produkt nach der allgemeinen Formel II kann dabei in reiner Form oder aber in Mischungen mit einer oder mehreren anderen der Komponenten aus Schritt 1 eingesetzt werden.

Die Hydrierung zum Produkt nach der allgemeinen Formel III kann als Flüssig- und/oder Gasphasenhydrierung oder in Kombination dieser Techniken erfolgen und sie kann in einem oder mehreren Schritten erfolgen wie beispielsweise in einer Vor- und einer Endhydrierung.

Die Hydrierung kann kontinuierlich oder diskontinuierlich durchgeführt werden. Als Reaktoren können die bekannten Standards für Hydrierungen eingesetzt werden, beispielsweise Rieselbett-Reaktoren (trickle bed). Die bei der Reaktion entstehende Reaktionswärme wird nach bekannten Verfahren abgeführt, beispielsweise durch interne oder externe Kühler. Konkret kann dies den Einsatz von Rohbündelreaktoren, Kühlfingern, Kühlschlangen oder - platten oder die Kühlung eines Rückführungsstroms (Reaktoren mit Kreislauf, Recycling), bedeuten.

Die Hydrierung wird katalysiert durchgeführt. Es können dabei sowohl homogene als auch heterogene Katalysatoren eingesetzt werden. Beispielsweise werden Übergangsmetalle als Katalysatoren für diese Hydrierung eingesetzt, insbesondere Kupfer, Chrom und die Metalle der VIII. Nebengruppe des Periodensystems.

Bei Einsatz von homogenen Katalysatoren werden neben dem Katalysatormetall zusätzliche Liganden eingesetzt. Geeignete Liganden sind beispielsweise Verbindungen des dreiwertigen Phosphors (zum Beispiel Phosphine oder Phosphite), Verbindungen des dreiwertigen Arsens oder Antimons, Stickstoffverbindungen (zum Beispiel Amine, Pyridine, Nitrile), Halogenide, Kohlenmonoxid und Cyanid.

Bei heterogenen Katalysatoren können die oben genannten Metalle mit anderen Metallen oder Moderatoren modifiziert sein. So werden zum Beispiel heterogene Palladiumkatalysatoren oftmals durch Zusatz von Schwefel oder Kohlenmonoxid in ihrer Aktivität und Selektivität modifiziert. Kupfer-Katalysatoren wird oftmals ein Anteil an Chrom zugesetzt.

Der Einsatz von geträgerten Katalysatoren ist in der Regel vorteilhaft, da geringere Metallmengen benötigt werden und über die Beschaffenheit des Trägers zusätzlich die Eigenschaften des Katalysators beeinflusst werden können. Als Trägermaterialien haben sich beispielsweise Aktivkohle, Aluminiumoxid, Siliziumdioxid, Silizium-Aluminium-Oxid, Bariumcarbonat, Bariumsulfat und Kieselgur bewährt.

Die Hydrierung des 2,7-Octadienylrestes zum 2-Octenylrest ist aus der Literatur bekannt. Beispiele für die homogen katalysierte Hydrierung finden sich in Chemistry Letters 1977, 1083 - 1084 und Bull. Chem. Soc. Jap. 1968, 41, 254 - 255. In US 5 118 837 wird der Einsatz heterogener Katalysatoren beschrieben.

Die Hydrierungen werden bei Temperaturen von 0 bis 400 °C, bevorzugt zwischen 20 und 200 °C durchgeführt. Der Druck liegt dabei zwischen 0,01 und 300 bar, bevorzugt zwischen 0,1 und 125 bar, besonders bevorzugt zwischen 1 und 64 bar.

Die Hydrierung in der Flüssigphase, egal ob homogen oder heterogen katalysiert, kann ohne oder in Gegenwart von zusätzlichen Lösemitteln durchgeführt werden. Beispiele für geeignete Lösemittel sind aliphatische und cycloaliphatische Kohlenwasserstoffe wie beispielsweise C₃-C₁₆-Alkane, Mischungen niederer oder höherer Alkane (C₃-C₂₀), Cyclohexan, Cyclooctan und Ethylcyclohexan; Alkohole wie beispielsweise Methanol, Ethanol, Propanol, Isopropanol, n-Butanol, Isobutanol, 2-Ethylhexanol, Isononanol und Isotridecanol; Polyole wie beispielsweise Ethylenglykol, Propylenglykol, 1,3-Propandiol und 1,4-Butandiol; Carbonsäureester wie beispielsweise Essigsäureethylester; Ether wie beispielsweise Dipropylether, Diethylether, Dimethylether, Methyl-tert.-butylether, Methyloctylether, 3-Methoxyoctan, Dioxan, Tetrahydrofuran, Alkylether von Ethylenglykol, Diethylenglykol und Polyethylenglykol; Sulfolan, Dimethylsulfoxid, Ethylencarbonat, Propylencarbonat und Wasser. Die Lösemittel kommen allein oder auch als Mischungen verschiedener Lösemittel zum Einsatz.

Bei der Hydrierung in der Flüssigphase können auch mehrere flüssige Phasen vorliegen. Dieses Verfahren ist besonders vorteilhaft, wenn Katalysator und Produkt in unterschiedlichen Phasen vorliegen, da dann der Katalysator einfach über eine Phasentrennung abgetrennt werden kann. Oftmals bildet dabei Wasser eine der flüssigen Phasen aus. Es werden beispielsweise aber auch perfluorierte Kohlenwasserstoffe, ionische Flüssigkeiten und überkritisches Kohlendioxid eingesetzt (zu Ionischen Flüssigkeiten vgl. P. Wasserscheid, W. Keim, Angew. Chem., Int. Ed. 2000, 39, 3772 - 3789). Einen Überblick über Wasser als Trägerphase für den Katalysator findet man beispielsweise in B. Cornils, W. A. Herrmann (Eds.) "Aqueous-Phase Organometallic Catalysis", Wiley-VCH, Weinheim, New-York, Chichester, Brisbane, Singapore, Toronto, 1998.

Bei den Hydrierungen in der Gashase können neben Wasserstoff und Substrat noch andere Gase anwesend sein. Beispielsweise können Stickstoff und/oder Argon, aber auch unter den Hydrierbedingungen gasförmige Alkane wie beispielsweise Methan, Propan oder Butan zugesetzt werden.

Für die Hydrierung in der Gasphase und in der Flüssigphase gilt, dass eine oder mehrere Komponenten aus Schritt 1 des erfindungsgemäßen Verfahrens vollständig oder teilweise zugegen sein können. Dabei kann es vorkommen, dass diese Komponenten unter den Bedingungen der Hydrierung ebenfalls reduziert werden. So werden beispielsweise die als Nebenprodukt entstehenden an 3-Position substituierten 1,7-Octadiene wenigstens teilweise hydriert, aus dem 1,3,7-Oetatrien werden ebenfalls wenigstens teilweise weniger ungesättigte oder gesättigte Spezies entstehen (Octadiene, Octene, Octan).

Die Hydrierung in Schritt 2 des erfindungsgemäßen Verfahrens kann kontinuierlich, semikontinuierlich oder diskontinuierlich (batchweise) erfolgen. Bevorzugt wird die kontinuierliche Verfahrensführung.

Vorzugsweise wird in Schritt 2 des erfindungsgemäßen Verfahrens ein möglichst vollständiger Umsatz der Verbindung nach der allgemeinen Formel II erreicht. Es ist aber auch möglich, die Reaktion nach einem Teilumsatz abzubrechen und die nicht umgesetzte Menge an II nach Abtrennung von den restlichen Komponenten in den Schritt 2 zurückzuführen oder gegebenenfalls anderweitig zu verwerten.

Das Produkt nach der allgemeinen Formel III aus Schritt 2 wird im dritten Schritt zu 1-Octen und weiteren Spaltprodukten umgesetzt. Hierzu ist es gegebenenfalls sinnvoll, das Produkt nach der allgemeinen Formel III vorher durch physikalische Verfahren aufzureinigen. Grundsätzlich können alle Verfahren oder Kombinationen von Verfahren zur Anwendung kommen, mit denen die Nebenprodukte ganz oder teilweise von der Verbindung nach der allgemeinen Formel III abgetrennt werden können. Bevorzugte Trenntechnik ist die Destillation. Für die destillative Trennung können alle verfügbaren Techniken eingesetzt werden, beispielsweise Bodenkolonnen, Kolonnen mit Packungen, Trennwandkolonnen, Extraktivdestillation, Dünnschichtverdampfer und Fallfilmverdampfer. Die destillative Trennung kann in einem oder in mehreren Schritten erfolgen und ist abhängig von den Siedepunkten der im Produktgemisch enthaltenen Komponenten.

In Schritt 3 des erfindungsgemäßen Verfahrens wird die Verbindung nach der allgemeinen Formel III gespalten, wobei 1-Octen entsteht. Die weiteren Spaltprodukte sind abhängig vom in Schritt 1 eingesetzten Telogen. Bei Einsatz von Methanol als Telogen in Schritt 1 des erfindungsgemäßen Verfahrens wird beispielsweise Formaldehyd gebildet, bei Einsatz von Ethanol Acetaldehyd, bei n-Butanol Butanal und bei Diethylamin Ethylethylidenamin.

Die Spaltung kann sowohl in der Flüssigphase als auch in der Gasphase durchgeführt werden. Die Spaltung kann in Gegenwart jeder Menge anderer Stoffe erfolgen, die unter den Spaltbedingungen inert beziehungsweise weitgehend inert sind. Beispielsweise können Stickstoff oder Argon, aber auch Wasser, Wasserdampf oder Alkane wie beispielsweise Methan, Propan oder Butan zugesetzt werden.

Die Temperatur, bei der die Spaltung der Verbindung nach der allgemeinen Formel III erfolgt, liegt zwischen 100 und 800 °C, bevorzugt zwischen 150 und 600 °C, besonders bevorzugt zwischen 250 und 500 °C.

Der Druck beträgt 0,05 bis 300 bar, bevorzugt 1 bis 125 bar, besonders bevorzugt 1 bis 64 bar.

Die Spaltungsreaktion kann ohne oder in Anwesenheit von heterogenen Katalysatoren durchgeführt werden. Bevorzugt eingesetzt werden Katalysatoren mit lewissauren Zentren wie beispielsweise amorphe Silika-Aluminas, Aluminas, Silikas, Kieselsäuren, aluminiumhaltige Kieselsäuren, Tonerden und Zeolithe.

Die Spaltung in Schritt 3 des erfindungsgemäßen Verfahren kann kontinuierlich, semikontinuierlich oder diskontinuierlich (batchweise) erfolgen.

Eine weitere Verfahrensvariante ist die Spaltung der Verbindung nach der allgemeinen Formel III bei gleichzeitiger Abtrennung der Spaltungsprodukte. Die Abtrennung der Spaltungsprodukte kann beispielsweise über die Gasphase erfolgen. Technisch ist dies zum Beispiel durch eine Destillation realisierbar; im unteren, wärmeren Teil der Destillation wird die Verbindung nach der allgemeinen Formel III gespalten, das entstehende 1-Octen und gegebenenfalls weitere Spaltungsprodukte werden über Kopf abgetrennt.

In der Spaltung wird die Verbindung nach der allgemeinen Formel III vollständig oder teilweise umgesetzt. Bei einem Teilumsatz enthält der Austrag der Spaltung noch nicht umgesetztes Edukt der allgemeinen Formel III. Dies kann, nach Abtrennung des gebildeten 1-Octens und gegebenenfalls anderer Spaltungsprodukte, in die Spaltung zurückgeführt werden. Es ist dabei auch möglich, nur das 1-Octen und gegebenenfalls einen Teil der Spaltprodukte abzutrennen und die Rückführung in die Vorreinigung vor der eigentlichen Spaltung zurückzuführen.

Die Abtrennung des 1-Octens von den anderen Komponenten des Austrags der Spaltung erfolgt nach bekannten Verfahren wie beispielsweise Phasenseparation, Extraktion, Wäsche, Destillation oder Fällung. Sie ist stark abhängig von dem in der Telomerisation eingesetzten Telogen. So kann der bei der Spaltung des 1-Methoxy-2-octens entstehende Formaldehyd einfach über eine Extraktion mit Wasser vom 1-Octen getrennt werden. Wird bei der Spaltung des 1-Methoxy-2-octens Wasser oder Wasserdampf zugesetzt, fällt bei der Aufarbeitung ebenfalls eine wässrige Formaldehydlösung an. In beiden Fällen kann dann die organische Phase, die das 1-Octen enthält, zum Beispiel über eine Destillation weiter aufgereinigt werden. Setzt man hingegen beispielsweise Butanol als Telogen im Telomerisationsschritt ein, dann wird bei der Spaltung des 1-Butoxy-2-octens unter anderem Butyraldehyd gebildet. In diesem Fall können die Produkte der Spaltung beispielsweise durch eine Destillation in die einzelnen Komponenten aufgetrennt werden.

Bei der Spaltung der Verbindung nach der allgemeinen Formel III entstehen neben dem 1-Octen auch andere Spaltprodukte mit ungesättigten Bindungen (Doppel- und/oder Dreifachbindungen), die in der vorliegenden Beschreibung als Spaltprodukte IV bezeichnet werden. Eine Option der vorliegenden Erfindung ist es, diese Spaltprodukte mit Wasserstoff zu hydrieren. Diese Hydrierung kann direkt während der Spaltung, im Anschluss an die Spaltung oder nach teilweiser oder vollständiger Auftrennung der Produkte aus Schritt 3 des erfindungsgemäßen Verfahrens erfolgen. Das Produkt der Hydrierung kann, gegebenenfalls nach einer Aufreinigung, ganz oder teilweise in Schritt 1 als Telogen eingesetzt werden. Wird beispielsweise Ethanol als Telogen eingesetzt, entsteht in Schritt 3 des erfindungsgemäßen Verfahrens Acetaldehyd, aus dem nach Hydrierung wieder Ethanol entsteht, aus Butanol wird entsprechend Butyraldehyd, der wieder zu Butanol hydriert werden kann, usw.

Die Hydrierung der Spaltprodukte IV wird katalysiert in einer oder mehreren Stufen durchgeführt. In den einzelnen Stufen kann die Hydrierung in der Gas- oder in der Flüssigphase erfolgen. Es können homogen gelöste oder heterogene Katalysatoren eingesetzt werden. Bevorzugt wird der Einsatz heterogener Katalysatoren. Beispielsweise werden Übergangsmetalle als Katalysatoren für die Hydrierung des Spaltprodukts IV eingesetzt. Insbesondere seien Kupfer, Chrom und die Metalle der VIII. Nebengruppe des Periodensystems genannt.

Bei heterogenen Katalysatoren können die oben genannten Metalle mit anderen Metallen oder Moderatoren modifiziert sein. Kupfer-Katalysatoren wird beispielsweise oft ein Anteil an Chrom zugesetzt.

Der Einsatz von geträgerten Katalysatoren ist in der Regel vorteilhaft, da geringere Metallmengen benötigt werden und über die Beschaffenheit des Trägers zusätzlich die Eigenschaften des Katalysators beeinflusst werden können. Als Trägermaterialien haben sich beispielsweise Aktivkohle, Aluminiumoxid, Siliziumdioxid, Silizium-Aluminium-Oxid, Bariumcarbonat, Bariumsulfat und/oder Kieselgur bewährt.

Die Hydrierungen der Spaltprodukte IV werden, wenn sie nicht unter den Bedingungen der Spaltung erfolgen, bei Temperaturen von 0 bis 400 °C, bevorzugt zwischen 50 und 250 °C durchgeführt. Der Druck liegt dabei zwischen 0,01 und 300 bar, bevorzugt zwischen 0,1 und 125 bar, besonders bevorzugt zwischen 1 und 64 bar.

Als Reaktoren können die bekannten Standards für Hydrierungen eingesetzt werden, beispielsweise Tricklebett-Reaktoren. Die bei der Reaktion entstehende Reaktionswärme wird nach bekannten Verfahren abgeführt, beispielsweise durch interne oder externe Kühler. Konkret kann dies den Einsatz von Rohrbündelreaktoren, Kühlfingern, Kühlschlangen oder - platten oder die Kühlung eines Rückführungsstroms (Reaktoren mit Kreislauf, Recycling), bedeuten.

Wird die Spaltung der Verbindungen nach der allgemeinen Formel III und die Hydrierung der Spaltprodukte IV in einem Schritt durchgeführt, können die bereits bei den jeweiligen Reaktionen eingesetzten heterogenen Katalysatoren nebeneinander eingesetzt werden. Es ist zudem möglich, Katalysatoren einzusetzen, die beide Reaktionen katalysieren, beispielsweise Übergangsmetalle auf lewissauren Trägem. In diesem Falle muss die Spaltungsreaktion in Gegenwart von Wasserstoff durchgeführt werden.

Die Hydrierung in der Flüssigphase, egal ob homogen oder heterogen katalysiert, kann ohne oder in Gegenwart von zusätzlichen Lösemitteln durchgeführt werden. Beispiele für geeignete Lösemittel sind Wasser, aliphatische und cycloaliphatische Kohlenwasserstoffe wie beispielsweise C₃-C₁₆-Alkane, Mischungen niederer oder höherer Alkane (C₃-C₂₀), Cyclohexan, Cyclooctan und Ethylcyclohexan; Alkohole wie beispielsweise Methanol, Ethanol, Propanol, Isopropanol, n-Butanol, Isobutanol, 2-Ethylhexanol, Isononanol und Isotridecanol; Polyole wie beispielsweise Ethylenglykol, Propylenglykol, 1,3-Propandiol und 1,4-Butandiol; Carbonsäureester wie beispielsweise Essigsäureethylester; Ether wie beispielsweise Dipropylether, Diethylether, Dimethylether, Methyl-tert.-butylether, Methyloctylether, 3-Methoxyoctan, Dioxan, Tetrahydrofuran, Alkylether von Ethylenglykol, Diethylenglykol und Polyethylenglykol; Sulfolan, Dimethylsulfoxid, Ethylencarbonat, Propylencarbonat und Wasser. Die Lösemittel kommen allein oder auch als Mischungen verschiedener Lösemittel zum Einsatz.

Bei der Spaltung können neben dem 1-Octen geringe Mengen anderer C₈-Olefine entsehen. So kann 2-Octen durch Isomerisierung des 1-Octens gebildet werden, 3-Octen kann aus dem 2-Octen entstehen usw. Auch Octan und Octadiene können gebildet werden. Zur Erreichung einer sehr hohen 1-Octen-Reinheit (> 97 %) kann es daher notwendig sein, einen Teil dieser C₈-Komponenten abzutrennen. Dies kann über eine destillative Aufreinigung erfolgen. Diese kann sowohl zusammen mit der Abtrennung anderer Produkte aus dem Spaltungsschritt (und optional der Hydrierungsprodukte der Spaltprodukte IV) erfolgen, oder separat als Aufreinigung eines zuvor isolierten C₈-Schnitts erfolgen.

Die folgenden Beispiele sollen die Erfindung erläutern, nicht aber ihren Anwendungsbereich beschränken, der sich aus der Beschreibung und den Patentansprüchen ergibt.

### Beispiele

### Beispiel 1 (Methanol als Telogen, 1-Methoxy 2,7-octadien)

In einem 701 Autoklav wurden 14 kg Methanol, 21 kg 1,3-Butadien, 7,5 g Palladium(II)-acetat, 85 g Triphenylphosphin und 160 g Triethylamin unter Ausschluss von Wasser und Sauerstoff auf 80 °C erhitzt. Dabei war ein Druckanstieg auf 8 bar zu beobachten. Unter diesen Bedingungen startete die Reaktion. Mit der Beginn der Umsetzung von 1,3-Butadien sank der Druck wieder ab. Nach 24 Stunden wurde der Autoklav auf Raumtemperatur abgekühlt und der Restdruck entspannt. Laut GC-Analyse hatte sich das 1,3-Butadien zu 98 % umgesetzt. Als Hauptprodukte wurden erhalten:

| **Komponente** | **CAS No.** | **% Anteil** |
|---|---|---|
| Methanol | 67-56-1 | 32,3 |
| 1,3,7-Octatrien | 1002-35-3 | 9,3 |
| 4-Vinylcyclohexen | 100-40-3 | 0,2 |
| 3-Methoxy-1,7-octadien | 20202-62-4 | 7,8 |
| 1-Methoxy-2,7-octadien | 14543-49-8 | 48,8 |
| Rest | | 1,6 |

Zur Aufarbeitung wurde der Reaktoraustrag bei 80 mbar in einer diskontinuierlichen Destillation in Katalysatorrückstand und Destillat aufgetrennt.

### Beispiel 2 (homogen katalysierte Hydrierung)

In einen 3-1 Büchi Autoklav wurden 1000 g 1-Methoxy-2,7-octadien, 500 ml Tetrahydrofuran, 500 ml Ethanol und 2,5 g Tris(triphenylphosphin)ruthenium(II)-chlorid, gegeben. Die Temperatur wurde auf 30 °C eingestellt und es wurde 30 bar Wasserstoffdruck aufgegeben. Die Reaktion wurde über die aufgenommene Menge an Wasserstoff und über GC-Analysen verfolgt. Nach 6 Stunden wurde die Reaktion abgebrochen. Laut GC-Analyse wurde das 1-Methoxy-2,7-octadien zu 98 % umgesetzt, mit einer Selektivität von 89 % zum 1-Methoxy-2-octen (cis und trans). Das 1-Methoxy-2-octen wurde destillativ von Lösemitteln und Katalysator abgetrennt.

### Beispiel 3 (heterogen katalysierte Hydrierung)

In einem gradientenfreien Differentialkreislaufreaktor der Firma Xytel wurden 15 g eines heterogenen Rutheniumkatalysators vorgelegt. Zur Immobilisierung befand sich der Katalysator in einem Körbchen aus Drahtgewebe. Der Gewichtsanteil des Rutheniums auf dem Trägermaterial γ-Al₂O₃ betrug 1 Gew-%. Vor Reaktionsbeginn wurde der Katalysator bei 200 °C in einer Wasserstoffatmosphäre reduziert. Nach der Reduktion wurden 900 ml eines Gemisches aus trans-1-Methoxy-2,7-octadien und cis-1-Methoxy-2,7-octadien im Massenverhältnis 96 : 4 dem Differentialkreislaufreaktor zugeführt. Bei einem Wasserstoffpartialdruck von 10 bar wurde das Reaktionsgemisch unter diskontinuierlichen Bedingungen hydriert. Gemäß GC-Analyse wurde bei 40 °C folgender Reaktionsfortschritt beobachtet:

| (cis-MOE = cis-1-Methoxy-2-octen, trans-MOE = trans-1-Methoxy-2-octen, cis-MODE = cis-1-Methoxy-2,7-octadien, trans-MODE = trans-1-Methoxy-2,7-octadien) | | | | | |
|---|---|---|---|---|---|
| LHSV⁻¹ kg h / Itr | cis-MOE Gew.-% | trans-MOE Gew.-% | cis-MODE Gew.-% | trans-MODE Gew.-% | Rest Gew.-% |
| 0,0083 | 0,13 | 1,33 | 6,25 | 91,25 | 1,04 |
| 0,0167 | 0,13 | 1,99 | 6,16 | 90,33 | 1,38 |
| 0,0250 | 0,18 | 3,05 | 6,20 | 88,40 | 2,17 |
| 0,0333 | 0,23 | 4,11 | 6,01 | 87,97 | 1,68 |
| 0,0417 | 0,33 | 5,57 | 5,95 | 86,12 | 2,04 |
| 0,0500 | 0,40 | 6,80 | 5,77 | 85,04 | 1,99 |
| 0,0667 | 0,50 | 8,71 | 5,60 | 82,11 | 3,08 |
| 0,0833 | 0,64 | 11,17 | 5,45 | 80,26 | 2,48 |
| 0,1083 | 0,81 | 13,87 | 5,24 | 77,39 | 2,69 |
| 0,1333 | 1,00 | 16,95 | 4,97 | 74,02 | 3,05 |
| 0,1583 | 1,18 | 19,87 | 4,74 | 70,87 | 3,35 |
| 0,3750 | 2,98 | 52,01 | 2,24 | 36,08 | 6,69 |
| 0,4000 | 3,26 | 57,11 | 1,86 | 30,09 | 7,69 |
| 0,4250 | 3,48 | 61,01 | 1,59 | 25,52 | 8,40 |
| 0,4500 | 3,67 | 65,01 | 1,28 | 21,55 | 8,49 |
| 0,4917 | 4,03 | 72,13 | 0,80 | 13,47 | 9,58 |
| 0,5250 | 4,20 | 76,2 | 0,54 | 8,67 | 10,39 |

Die oben beschriebene Versuchsreihe wurde unter gleichen Versuchsbedingungen auch bei 50°C durchgeführt. Erwartungsgemäß steigt die Reaktionsgeschwindigkeit mit der Temperatur. Gemäß GC-Analyse wurde bei 50 °C folgender Reaktionsfortschritt beobachtet.

| LHSV⁻¹ kg h / Itr | cis-MOE Gew.-% | trans-MOE Gew.-% | cis-MODE Gew.-% | t-MODE Gew.-% | Rest Gew.-% |
|---|---|---|---|---|---|
| 0,0083 | 0,12 | 2,77 | 3,80 | 90,33 | 2,99 |
| 0,0167 | 0,16 | 4,16 | 3,77 | 89,14 | 2,76 |
| 0,0250 | 0,23 | 6,01 | 3,71 | 87,20 | 2,85 |
| 0,0333 | 0,31 | 7,83 | 3,64 | 84,24 | 3,98 |
| 0,0417 | 0,36 | 9,01 | 3,63 | 83,13 | 3,87 |
| 0,0583 | 0,48 | 11,84 | 3,55 | 79,63 | 4,50 |
| 0,0917 | 0,70 | 17,08 | 3,31 | 73,20 | 5,70 |
| 0,1167 | 0,88 | 21,37 | 3,16 | 68,21 | 6,38 |
| 0,3667 | 3,06 | 64,30 | 1,09 | 17,87 | 13,68 |
| 0,3833 | 3,28 | 67,72 | 0,88 | 13,97 | 14,15 |
| 0,4000 | 3,44 | 70,27 | 0,72 | 10,82 | 14,75 |
| 0,4167 | 3,59 | 71,53 | 0,71 | 8,01 | 16,16 |
| 0,4333 | 1,28 | 74,29 | 0,44 | 5,52 | 18,48 |
| 0,4667 | 3,91 | 75,77 | 0,30 | 1,51 | 18,51 |

### Beispiel 4 (Spaltung zum 1-Octen)

In einen 100 ml gradientenfreien Differentialkreislaufreaktor wurde kontinuierlich ein gasförmiges Gemisch aus 1-Methoxy-2-octen (cis und trans) [CAS 60171-33-7] und Stickstoff zugeführt. Die zugeführte Gesamtmenge betrug 60 Nml/min. Der Inertgasanteil im Zulauf lag bei 83 %. Unter atmosphärischem Druck und in einem Temperaturintervall von 375 - 450 °C wurde die Spaltung des 1-Methoxy 2-octens zu 1-Octen und Formaldehyd durchgeführt. Bezogen auf das 1-Methoxy-2-octen im Eingangsgas wurden bei einer Verweilzeit von 40 s folgende Umsatzraten von 1-Methoxy-2-octen bzw. Selektivitäten zum 1-Octen beobachtet:

| Temperatur [°C] | 375 | 400 | 425 |
|---|---|---|---|
| Umsatz Methyl-2-octenylether [%] | 4.83 | 16.7 | 36.7 |
| Selektivität Bildung 1-Octen [%] | 89.7 | 77.7 | 75.7 |

## Patentansprüche

1. Verfahren zur Herstellung von 1-Octen, wobei
(I) 1,3-Butadien in Gegenwart eines Telomerisationskatalysators mit einem Telogen der allgemeinen Formel H-X-Y-H, worin X für O, N, S oder P und Y für C, N oder Si steht und X und Y je nach Wertigkeit weitere Substituenten tragen, zu einem Telomer der allgemeinen Formel H₂C=CH-CH₂-CH₂-CH₂-CH-CH-CH₂-X-Y-H umgesetzt wird, **dadurch gekennzeichnet, dass**
(II) das Telomer zu einem 1-substituierten 2-Octen der Formel H₃C-CH₂-CH₂-CH₂-CH₂-CH=CH-CH₂-X-Y-H hydriert wird und
(III) das 1-substituierte 2-Octen zum 1-Octen gespalten wird.

2. Verfahren zur Herstellung von 1-Octen nach Anspruch 1,
**dadurch gekennzeichnet,**
**dass** die Substituenten an X und/oder Y Wasserstoff, Alkylreste mit 1 - 50 Kohlenstoffatomen, Arylreste mit 6 - 50 Kohlenstoffatomen und/oder Heteroarylreste sind, wobei diese Substituenten jeweils gleich oder verschieden sind und ihrerseits wiederum mit den Gruppen Alkyl, Aryl, -F, -Cl, -Br, -I, -CF₃, -OR, -COR, -CO₂R, -OCOR, -SR, -SO₂R, -SOR, -SO₃R, -SO₂NR₂, -NR₂, -N=CR₂, -NH₂, mit R = H, substituierter oder unsubstituierter, aliphatischer oder aromatischer Kohlenwasserstoffrest mit 1 bis 25 Kohlenstoffatomen, substituiert sein können.

3. Verfahren zur Herstellung von 1-Octen nach Anspruch 1 oder 2,
**dadurch gekennzeichnet,**
**dass** als Telogen ein Monoalkohol, ein Dialkohol, ein primäres Amin oder ein sekundäres Amin eingesetzt wird.

4. Verfahren zur Herstellung von 1-Octen nach einem der Ansprüche 1 bis 3,
**dadurch gekennzeichnet,**
**dass** als Telogen Methanol, Ethanol, n-Propanol, Isopropanol, Allylalkohol, n-Butanol, i-Butanol, Octanol, 2-Ethylhexanol, Isononanol, Benzylalkohol, Cyclohexanol, Cyclopentanol, 2,7-Octadien-1-ol, Ethylenglykol, 1,2-Propandiol, 1,3-Propandiol, 1,4-Butandiol, 1,2-Butandiol, 2,3-Butandiol, 1,3-Butandiol oder α-Hydroxyessigsäureester eingesetzt wird.

5. Verfahren zur Herstellung von 1-Octen nach einem der Ansprüche 1 bis 3,
**dadurch gekennzeichnet,**
**dass** als Telogen Methylamin, Ethylamin, Propylamin, Butylamin, Octylamin, 2,7-Octadienylamin, Dodecyclamin, Ethylendiamin, Hexamethylendiamin, Dimethylamin, Diethylamin, N-Methylanilin, Bis(2,7-Octadienyl)amin, Dicyclohexylamin, Methylcyclohexylamin, Pyrrolidin, Piperidin, Morpholin, Piperazin oder Hexamethylenimin eingesetzt wird.

6. Verfahren zur Herstellung von 1-Octen nach mindestens einem der Ansprüche 1 bis 5,
**dadurch gekennzeichnet,**
**dass** in Schritt 1 pro Mol aktivem Wasserstoffatom des Telogens 0,001 Mol bis 10 Mol 1,3-Butadien eingesetzt werden.

7. Verfahren zur Herstellung von 1-Octen nach mindestens einem der Ansprüche 1 bis 6,
**dadurch gekennzeichnet,**
**dass** in Schritt 1 nicht abreagiertes Telogen zurückgewonnen und in Schritt 1 zurückgeführt wird.

8. Verfahren zur Herstellung von 1-Octen nach mindestens einem der Ansprüche 1 bis 7,
**dadurch gekennzeichnet,**
**dass** in Schritt 1 ein Übergangsmetall der VIII. Nebengruppe des Periodensystems als Katalysatormetall eingesetzt wird.

9. Verfahren zur Herstellung von 1-Octen nach Anspruch 8,
**dadurch gekennzeichnet,**
**dass** in Schritt 1 Palladium als Katalysatormetall eingesetzt wird.

10. Verfahren zur Herstellung von 1-Octen nach mindestens einem der vorhergehenden Ansprüche,
**dadurch gekennzeichnet,**
**dass** in Schritt 1 eine Verbindung mit einem trivalenten Phosphor-, Arsen-, Antimon- oder Stickstoffatom als Ligand eingesetzt wird.

11. Verfahren zur Herstellung von 1-Octen nach Anspruch 10,
**dadurch gekennzeichnet,**
**dass** in Schritt 1 ein Phosphin, Phosphit, Phosphonit oder Phosphinit als Ligand eingesetzt wird.

12. Verfahren zur Herstellung von 1-Octen nach mindestens einem der Ansprüche 1 bis 11,
**dadurch gekennzeichnet,**
**dass** in Schritt 1 zusätzlich eine Base eingesetzt wird.

13. Verfahren zur Herstellung von 1-Octen nach mindestens einem der Ansprüche 1 bis 12,
**dadurch gekennzeichnet,**
**dass** der Telomerisationskatalysator von Schritt 1 zurückgewonnen und ganz oder teilweise in den Prozess zurückgeführt wird.

14. Verfahren zur Herstellung von 1-Octen nach mindestens einem der Ansprüche 1 bis 12,
**dadurch gekennzeichnet,**
**dass** der Telomerisationskatalysator von Schritt 1 nicht in Schritt 1 zurückgeführt wird.

15. Verfahren zur Herstellung von 1-Octen nach Anspruch 1,
**dadurch gekennzeichnet,**
**dass** in Schritt 2 Wasserstoff und ein homogener Hydrierkatalysator eingesetzt werden.

16. Verfahren zur Herstellung von 1-Octen nach Anspruch 1,
**dadurch gekennzeichnet,**
**dass** in Schritt 2 Wasserstoff und ein heterogener Hydrierkatalysator eingesetzt wird.

17. Verfahren zur Herstellung von 1-Octen nach Anspruch 15 oder 16,
**dadurch gekennzeichnet,**
**dass** in Schritt 2 ein Hydrierkatalysator eingesetzt wird, der Kupfer, Chrom und/oder ein Übergangsmetall der VIII. Nebengruppe des Periodensystems enthält.

18. Verfahren zur Herstellung von 1-Octen nach mindestens einem der vorhergehenden Ansprüche,
**dadurch gekennzeichnet,**
**dass** in Schritt 2 das in Schritt 1 als Nebenprodukt anfallendes 1,3,7-Octatrien ganz oder teilweise hydriert wird.

19. Verfahren zur Herstellung von 1-Octen nach Anspruch 1,
**dadurch gekennzeichnet,**
**dass** in Schritt 2 das in Schritt 1 als Nebenprodukt anfallende an 3-Position substituierte 1,7-Octadien ganz oder teilweise hydriert wird.

20. Verfahren zur Herstellung von 1-Octen nach Anspruch 1,
**dadurch gekennzeichnet,**
**dass** die Spaltung in Schritt 3 bei Temperaturen zwischen 150 °C und 600 °C erfolgt.

21. Verfahren zur Herstellung von 1-Octen nach Anspruch 1,
**dadurch gekennzeichnet,**
**dass** im Zulauf zur Spaltung in Schritt 3 weniger als 5 % C₈-Kohlenwasserstoffe enthalten sind.

22. Verfahren zur Herstellung von 1-Octen nach Anspruch 1,
**dadurch gekennzeichnet,**
**dass** in Schritt 3 das an der 1-Position substituierte 2-Octen nur teilweise umgesetzt wird.

23. Verfahren zur Herstellung von 1-Octen nach Anspruch 1,
**dadurch gekennzeichnet,**
**dass** das nicht umgesetzte an der 1-Position substituierte 2-Octen von den restlichen Komponenten des Austrags aus Schritt 3 abgetrennt und ganz oder teilweise in Schritt 3 zurückgeführt wird.

24. Verfahren nach einem der vorhergehenden Ansprüche,
**dadurch gekennzeichnet,**
**dass** die Rückführung des an der 1-Position substituierten 2-Octens in eine dem Schritt 2 vorgeschaltete Reinigungsstufe erfolgt.

25. Verfahren zur Herstellung von 1-Octen nach einem der vorhergehenden Ansprüche,
**dadurch gekennzeichnet,**
**dass** die Spaltprodukte IV mit Wasserstoff hydriert werden, wobei die Hydrierung direkt während der Spaltung, im Anschluss an die Spaltung oder nach teilweiser oder vollständiger Auftrennung der Produkte aus Schritt 3 des Verfahrens erfolgt.

26. Verfahren zur Herstellung von 1-Octen nach einem der vorhergehenden Ansprüche,
**dadurch gekennzeichnet,**
**dass** in Schritt 3 die Spaltung zum 1-Octen gleichzeitig mit einer Abtrennung des 1-Octens und der gebildeten Spaltprodukte IV von den Edukten erfolgt.

27. Verfahren zur Herstellung von 1-Octen nach einem der vorhergehenden Ansprüche,
**dadurch gekennzeichnet,**
**dass** die Spaltung in Schritt 3 in Gegenwart von Wasser oder Wasserdampf durchgeführt wird.

28. Verfahren zur Herstellung von 1-Octen nach einem der vorhergehenden Ansprüche,
**dadurch gekennzeichnet,**
**dass** die Spaltung in Schritt 3 in Gegenwart von Katalysatoren durchgeführt wird.

## Claims

1. A process for preparing 1-octene, in which
(I) 1,3-butadiene is reacted with a telogen of the formula H-X-Y-H, where X is O, N, S or P and Y is C, N or Si and X and Y bear, depending on their valence, further substituents, in the presence of a telomerization catalyst to form a telomer of the formula H₂C=CH-CH₂-CH₂-CH₂-CH=CH-CH₂-X-Y-H, **characterized in that**
(II) the telomer is hydrogenated to form a 1-substituted 2-octene of the formula H₃C-CH₂-CH₂-CH₂-CH₂-CH=CH-CH₂-X-Y-H and
(III)the 1-substituted 2-octene is dissociated to give 1-octene.

2. A process for preparing 1-octene according to claim 1, **characterized in that** the substituents on X and/or Y are hydrogen, alkyl radicals having 1 - 50 carbon atoms, aryl radicals having 6 - 50 carbon atoms and/or heteroaryl radicals, the substituents being identical or different and optionally in turn substituted by the groups alkyl, aryl, -F, -Cl, -Br, -I, -CF₃, -OR, -COR, -CO₂R, -OCOR, -SR, -SO₂R, -SOR, -SO₃R, -SO₂NR₂, -NR₂, -N=CR₂, -NH₂ where R = H or a substituted or unsubstituted, aliphatic or aromatic hydrocarbon radical having from 1 to 25 carbon atoms.

3. A process for preparing 1-octene according to claim 1 or 2, **characterized in that** the telogen used is a monoalcohol, a dialcohol, a primary amine or a secondary amine.

4. A process for preparing 1-octene according to any one of claims 1 to 3, **characterized in that** the telogen used is methanol, ethanol, n-propanol, isopropanol, allyl alcohol, n-butanol, i-butanol, octanol, 2-ethylhexanol, isononanol, benzyl alcohol, cyclohexanol, cyclopentanol, 2,7-octadien-1-ol, ethylene glycol, 1,2-propanediol, 1,3-propanediol, 1,4-butanediol, 1,2-butanediol, 2,3-butanediol, 1,3-butanediol or an α-hydroxyacetic ester.

5. A process for preparing 1-octene according to any one of claims 1 to 3, **characterized in that** the telogen used is methylamine, ethylamine, propylamine, butylamine, octylamine, 2,7-octadienylamine, dodecylamine, ethylenediamine, hexamethylenediamine, dimethylamine, diethylamine, N-methylaniline, bis(2,7-octadienyl)amine,-dicyclohexylamine, methylcyclohexylamine, pyrrolidine, piperidine, morpholine, piperazine or hexamethylenimine.

6. A process for preparing 1-octene according to at least one of claims 1 to 5, **characterized in that** from 0.001 mol to 10 mol of 1,3-butadiene are used in step 1 for every mole of active hydrogen atom.

7. A process for preparing 1-octene according to at least one of claims 1 to 6, **characterized in that** telogen which has not reacted in step 1 is recovered and returned to step 1.

8. A process for preparing 1-octene according to at least one of claims 1 to 7, **characterized in that** a transition metal of transition group VIII of the Periodic Table is used as catalyst metal in step 1.

9. A process for preparing 1-octene according to claim 8, **characterized in that** palladium is used as catalyst metal in step 1.

10. A process for preparing 1-octene according to at least one of the preceding claims, **characterized in that** a compound containing a trivalent phosphorus, arsenic, antimony or nitrogen atom is used as ligand in step 1.

11. A process for preparing 1-octene according to claim 10, **characterized in that** a phosphine, phosphite, phosphonite or phosphinite is used as ligand in step 1.

12. A process for preparing 1-octene according to at least one of claims 1 to 11, **characterized in that** a base is additionally used in step 1.

13. A process for preparing 1-octene according to at least one of claims 1 to 12, **characterized in that** the telomerization catalyst of step 1 is recovered and returned in full or in part to the process.

14. A process for preparing 1-octene according to at least one of claims 1 to 12, **characterized in that** the telomerization catalyst of step 1 is not returned to step 1.

15. A process for preparing 1-octene according to claim 1, **characterized in that** hydrogen and a homogeneous hydrogenation catalyst are used in step 2.

16. A process for preparing 1-octene according to claim 1, **characterized in that** hydrogen and a heterogeneous hydrogenation catalyst are used in step 2.

17. A process for preparing 1-octene according to claim 15 or 16, **characterized in that** a hydrogenation catalyst comprising copper, chromium and/or a transition metal of transition group VIII of the Periodic Table is used in step 2.

18. A process for preparing 1-octene according to at least one of the preceding claims, **characterized in that** all or part of the 1,3,7-octatriene formed as by-product in step 1 is hydrogenated in step 2.

19. A process for preparing 1-octene according to claim 1, **characterized in that** all or part of the 1,7-octadiene substituted in the 3 position which is obtained as by-product in step 1 is hydrogenated in step 2.

20. A process for preparing 1-octene according to claim 1, **characterized in that** the dissociation in step 3 is carried out at temperatures in the range from 150°C to 600°C.

21. A process for preparing 1-octene according to claim 1, **characterized in that** less than 5% of C₈-hydrocarbons are present in the feed to the dissociation in step 3.

22. A process for preparing 1-octene according to claim 1, **characterized in that** the 2-octene substituted in the 1 position is only partly converted in step 3.

23. A process for preparing 1-octene according to claim 1, **characterized in that** the unreacted 2-octene substituted in the 1 position is separated off from the remaining components of the output from step 3 and is recirculated in full or in part to step 3.

24. A process according to any one of the preceding claims, **characterized in that** the 2-octene substituted in the 1 position is recirculated to a purification step which precedes step 2.

25. A process for preparing 1-octene according to any one of the preceding claims, **characterized in that** the dissociation products IV are hydrogenated by means of hydrogen either directly during the dissociation, subsequent to the dissociation or after partial or complete separation of the products from step 3 of the process.

26. A process for preparing 1-octene according to any one of the preceding claims, **characterized in that**, in step 3, the dissociation to give 1-octene is carried out simultaneously with separation of the 1-octene and the dissociation products IV formed from the starting materials.

27. A process for preparing 1-octene according to any one of the preceding claims, **characterized in that** the dissociation in step 3 is carried out in the presence of water or water vapour.

28. A process for preparing 1-octene according to any one of the preceding claims, **characterized in that** the dissociation in step 3 is carried out in the presence of catalysts.

## Revendications

1. Procédé de fabrication de 1-octène, dans lequel
(I) la réaction de 1,3-butadiène avec un télogène de formule générale H-X-Y-H, dans laquelle X représente O, N, S ou P et Y représente C, N ou Si et X et Y portent, indépendamment de leurs valences, d'autres substituants, en présence d'un catalyseur de télomérisation pour former un télomère de formule H₂C=CH-CH₂-CH₂-CH₂-CH=CH-CH₂-X-Y-H,
**caractérisé en ce que**
(II) le télomère est hydrogéné pour former un 2-octène 1-substitué de formule H₃C-CH₂-CH₂-CH₂-CH₂-CH=CH-CH₂-X-Y-H et
(III) le 2-octène 1-substitué est dissocié pour permettre l'obtention de 1-octène.

2. Procédé de fabrication de 1-octène selon la revendication 1,
**caractérisé en ce que**
les substituants sur X et/ou Y sont un atome d'hydrogène, des restes alkyles ayant 1 à 50 atomes de carbone, des restes aryles ayant 6 à 50 atomes de carbone et/ou des restes hétéroaryles, dans lesquels ces substituants sont à chaque fois identiques ou différents et peuvent être à leur tour substitués par les groupes alkyle, aryle, -F, -Cl, -Br, -I, -CF₃, -OR, -COR, -CO₂R, -OCOR, -SR, -SO₂R, -SOR, -SO₃R, -SO₂NR₂, -NR₂, -N=CR₂, -NH₂ avec R = H ou un reste hydrocarbure substitué ou non substitué, aliphatique ou aromatique, peuvent présenter de 1 à 25 atomes de carbone.

3. Procédé de fabrication de 1-octène selon la revendication 1 ou 2,
**caractérisé en ce que**
le télogène utilisé est un monoalcool, un dialcool, une amine primaire ou une amine secondaire.

4. Procédé de fabrication de 1-octène selon l'une quelconque des revendications 1 à 3,
**caractérisé en ce que**
le télogène utilisé est un méthanol, un éthanol, un n-propanol, un isopropanol, un alcool allylique, un n-butanol, un i-butanol, un octanol, un 2-éthylhexanol, un isononanol, un alcool benzylique, un cyclohexanol, un cyclopentanol, un 2,7-octadièn-1-ol, un éthylène glycol, un 1,2-propanediol, un 1,3-propanediol, un 1,4-butanediol, un 1,2-butanediol, un 2,3-butanediol, un 1,3-butanediol ou un ester α-hydroxyacétique.

5. Procédé de fabrication de 1-octène selon l'une quelconque des revendications 1 à 3,
**caractérisé en ce que**
le télogène utilisé est une méthylamine, une éthylamine, une propylamine, une butylamine, une octylamine, une 2,7-octadiénylamine, une dodécylamine, une éthylènediamine, une hexaméthylènediamine, une diméthylamine, une diéthylamine, une N-méthylaniline, une bis(2,7-octadiényl)amine, une dicyclohexylamine, une méthylcyclohexylamine, une pyrrolidine, une pipéridine, une morpholine, une pipérazine ou une hexaméthylènimine.

6. Procédé de fabrication de 1-octène selon l'une quelconque au moins des revendications 1 à 5,
**caractérisé en ce que**
de 0,001 mole à 10 moles de 1,3-butadiène sont utilisées dans l'étape 1 pour toute mole d'atome d'hydrogène active.

7. Procédé de fabrication de 1-octène selon l'une quelconque au moins des revendications 1 à 6,
**caractérisé en ce que**
le télogène qui est inaltéré dans l'étape 1 est récupéré et réintroduit dans l'étape 1.

8. Procédé de fabrication de 1-octène selon l'une quelconque au moins des revendications 1 à 7,
**caractérisé en ce qu'**
un métal de transition du groupe secondaire VIII du tableau périodique est utilisé comme métal catalytique dans l'étape 1.

9. Procédé de fabrication de 1-octène selon la revendication 8,
**caractérisé en ce que**
le palladium est utilisé comme métal catalytique dans l'étape 1.

10. Procédé de fabrication de 1-octène selon l'une quelconque au moins des revendications précédentes,
**caractérisé en ce qu'**
un composé contenant un atome de phosphore, d'arsenic, d'antimoine ou d'azote trivalent est utilisé comme coordinat dans l'étape 1.

11. Procédé de fabrication de 1-octène selon la revendication 10,
**caractérisé en ce qu'**
une phosphine, un phosphite, un phosphonite ou un phosphinite est utilisé comme coordinat dans l'étape 1.

12. Procédé pour préparer 1-octène selon l'une quelconque au moins des revendications 1 à 11,
**caractérisé en ce qu'**
une base est en plus utilisée dans l'étape 1.

13. Procédé de fabrication de 1-octène selon l'une quelconque au moins des revendications 1 à 12,
**caractérisé en ce que**
le catalyseur de télomérisation de l'étape 1 est récupéré et réintroduit en partie ou intégralement dans le procédé.

14. Procédé de fabrication de 1-octène selon l'une quelconque au moins des revendications 1 à 12,
**caractérisé en ce que**
le catalyseur de télomérisation de l'étape 1 n'est pas réintroduit dans l'étape 1.

15. Procédé de fabrication de 1-octène selon la revendication 1,
**caractérisé en ce que**
l'atome d'hydrogène et un catalyseur homogène d'hydrogénation sont utilisés dans l'étape 2.

16. Procédé de fabrication de 1-octène selon la revendication 1,
**caractérisé en ce que**
l'atome d'hydrogène et un catalyseur d'hydrogène hétérogène sont utilisés dans l'étape 2.

17. Procédé de fabrication de 1-octène selon la revendication 15 ou 16,
**caractérisé en ce qu'**
un catalyseur d'hydrogénation comprend le cuivre, le chrome et/ou un métal de transition du groupe secondaire VIII du tableau périodique qui est utilisé dans l'étape 2.

18. Procédé de fabrication de 1-octène selon l'une quelconque au moins des revendications précédentes,
**caractérisé en ce que**
tout ou partie du 1,3,7-octatriène formé comme produit secondaire dans l'étape 1 est hydrogéné dans l'étape 2.

19. Procédé de fabrication de 1-octène selon la revendication 1,
**caractérisé en ce que**
tout ou partie du 1,7-octadiène substitué en position 3 qui est obtenu comme produit secondaire dans l'étape 1 est hydrogéné dans l'étape 2.

20. Procédé de fabrication de 1-octène selon la revendication 1,
**caractérisé en ce que**
la séparation dans l'étape 3 est réalisée à des températures s'inscrivant dans une plage entre 150°C et 600°C.

21. Procédé de fabrication de 1-octène selon la revendication 1,
**caractérisé en ce que** moins de 5 % d'hydrocarbure en C₈ sont présents pour alimenter la séparation de l'étape 3.

22. Procédé de fabrication de 1-octène selon la revendication 1,
**caractérisé en ce que**
le 2-octène substitué en position 1 est seulement converti en partie en position 3.

23. Procédé de fabrication de 1-octène selon la revendication 1,
**caractérisé en ce que**
le 2-octène substitué inaltéré en position 1 est séparé des composants restants à la sortie de l'étape 3 et réinjecté en partie ou intégralement dans l'étape 3.

24. Procédé selon l'une quelconque des revendications précédentes,
**caractérisé en ce que**
le 2-octène substitué en position 1 est réinjecté dans une étape de purification qui précède l'étape 2.

25. Procédé de fabrication de 1-octène selon l'une quelconque des revendications précédentes,
**caractérisé en ce que**
les produits de séparation IV sont hydrogénés au moyen d'un atome d'hydrogène soit directement durant la séparation soit après la séparation ou après séparation partielle ou complète des produits de l'étape 3 du procédé.

26. Procédé de fabrication de 1-octène selon l'une quelconque des revendications précédentes,
**caractérisé en ce que**
dans l'étape 3, la séparation permettant d'obtenir 1-octène est réalisée simultanément à la séparation du 1-octène et les produits de séparation IV sont formés à partir des matériaux de départ.

27. Procédé de fabrication de 1-octène selon l'une quelconque des revendications précédentes,
**caractérisé en ce que**
la séparation de l'étape 3 est réalisée en présence d'eau ou de vapeur d'eau.

28. Procédé de fabrication de 1-octène selon l'une quelconque des revendications précédentes,
**caractérisé en ce que**
la séparation de l'étape 3 est réalisée en présence de catalyseurs.
